(19) Europäisches Patentamt
European Patent Office
Office européen des brevets

(11) Veröffentlichungsnummer: **0 012 291**
**A1**

# (12) EUROPÄISCHE PATENTANMELDUNG

(21) Anmeldenummer: **79104802.8**

(22) Anmeldetag: **01.12.79**

(51) Int. Cl.³: **C 07 D 405/14**, C 07 D 405/04 // C07D403/12, C07D249/06

(30) Priorität: **13.12.78 DE 2853765**

(43) Veröffentlichungstag der Anmeldung: **25.06.80 Patentblatt 80/13**

(84) Benannte Vertragsstaaten: **CH DE FR GB IT**

(71) Anmelder: **BAYER AG, Zentralbereich Patente, Marken und Lizenzen, D-5090 Leverkusen 1, Bayerwerk (DE)**

(72) Erfinder: **Wehling, Bernhard, Dr., Andreas-Gryphius-Strasse 26, D-5000 Köln 80 (DE)**
Erfinder: **Bremen, Josef, Dr., Am Kettnersbusch 1, D-5090 Leverkusen 3 (DE)**
Erfinder: **Schellhammer, Carl-Wolfgang, Dr., Katharinenthal 26, D-5060 Bergisch-Gladbach 2 (DE)**

(54) **Verfahren zur Herstellung von Benzimidazolylbenzofuranen.**

(57) Optische Aufheller der Formel

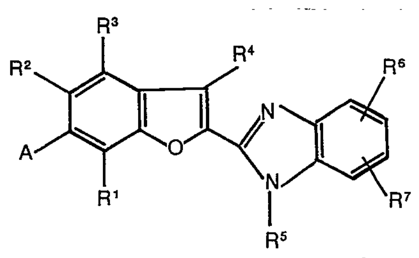

in der

| | |
|---|---|
| $R^1, R^2, R^3, R^7$ | Wasserstoff, Alkyl, Alkoxy oder Halogen |
| $R^4$ | Wasserstoff, Aryl oder Alkyl |
| $R^5$ | Wasserstoff oder Kohlenwasserstoffrest |
| $R^6$ | Wasserstoff, Halogen, Alkyl, Alkoxy u.a. |
| A | $R^1$ oder Aryl oder Rest der Formel |

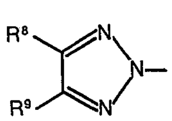

bedeuten, wobei
$R^8$ =Aryl, Alkyl, Aralkyl, Styryl oder Alkoxy,
$R^9$ =Wasserstoff, CN, COOR, CONRR' u.a.
R =Alkyl und
R' =R oder H,
erhält man auf einfache Weise, indem man Verbindungen der Formel

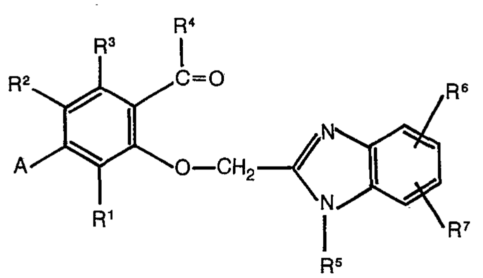

mit stark basischen Kondensationsmitteln, wie z. B. Alkalihydroxiden und -alkoholaten, ringschließend umsetzt.

Das Verfahren kann besonders vorteilhaft als Eintopfreaktion durchgeführt werden.

EP 0 012 291 A1

BAYER AKTIENGESELLSCHAFT 5090 Leverkusen, Bayerwerk
Zentralbereich K-by
Patente, Marken und Lizenzen

## Verfahren zur Herstellung von Benzimidazolylbenzofuranen

Die Erfindung betrifft ein Verfahren zur Herstellung von 2-[Benzimidazol-2-yl]-benzofuranen der allgemeinen Formel

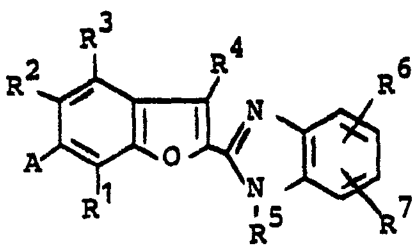

(I)

in der

$R^1, R^2, R^3$ Wasserstoff, Alkyl, Alkoxy oder Halogen,

$R^4$ Wasserstoff, Aryl, Alkyl,

$R^5$ Wasserstoff, Alkyl, Cycloalkyl, Alkenyl, Aryl oder Aralkyl,

$R^6$ Wasserstoff, Halogen, Alkyl, Alkoxy, Alkylsulfonyl, Phenylsulfonyl, CN, $CF_3$, COOR, $SO_3R$, CONRR' oder $SO_2NRR'$,

$R^7$ Wasserstoff, Halogen, Alkyl, Alkoxy,

A Wasserstoff, Halogen, Alkyl, Alkoxy oder Aryl oder einen Substituenten der allgemeinen Formel

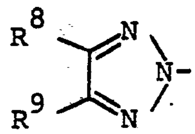

in der

$R^8$ Aryl, Alkyl, Aralkyl, Styryl oder Alkoxy,

$R^9$ Wasserstoff, CN, COOR, CONRR' oder $R^8$,

$R^8$ und $R^9$ zusammen einen annellierten hydroaromatischen oder aromatischen Ring,

R Alkyl und

R' R oder H bedeuten, wobei die vorstehend genannten Kohlenwasserstoff- und Alkoxyreste sowie die gegebenenfalls annellierten Ringsysteme durch in der Aufhellerchemie übliche Substituenten substituiert sein können.

Geeignete beliebige Alkyl- und Alkoxyreste haben 1-4 C-Atome.

Geeignete Arylreste sind Phenylreste. Geeignete nichtionische Substituenten sind Alkyl, Alkoxy, Halogen, CN, COOR, $SO_2R$ u.a.m. Unter "Halogen" wird vor allem Brom und insbesondere Chlor verstanden.

Das Verfahren ist dadurch gekennzeichnet, daß man eine Verbindung der Formel

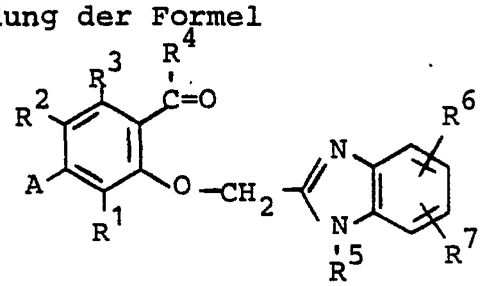

(II)

in der

$R^1, R^2, R^3, R^4, R^5, R^6, R^7$ und A die oben angegebene Bedeutung haben,

mit einem stark basischen Kondensationsmittel umsetzt. Die Kondensation erfolgt bei Temperaturen zwischen 0 und 100°C, bevorzugt bei 10-40°C. Vorteilhafterweise wird die Kondensation in einem organischen Lösungsmittel durchgeführt. Als Lösungsmittel eignen sich Alkohole wie z.B. Methanol, Äthanol oder Äthylenglykolmonomethyläther, Fettsäureamide wie Dimethylformamid oder Dimethylacetamid, Sulfoxide wie Dimethylsulfoxid und Hexamethylphosphorsäuretriamid.

Bevorzugte Kondensationsmittel sind Alkali- und Erdalkalihydroxyde wie Natriumhydroxyd und Kaliumhydroxyd, Alkalisalze von Alkoholen wie Natriummethylat, Natriumäthylat und Kalium-tert.-butylat, quartäre Ammoniumhydroxyde sowie stark basische Ionenaustauscher.

Die einzusetzende Kondensationsmittelmenge bewegt sich in weiten Grenzen. Obwohl für die Kondensationsreaktion im Lösungsmittel an sich eine katalytische Menge ausreichend ist, verwendet man sie jedoch vorteilhaft in der äquivalenten Menge, darüber hinaus jedoch auch in einer vielfach äquivalenten Menge, letzteres insbesondere dann, wenn es sich bei den zu kondensierenden Verbindungen um solche handelt, die hydrolysierbare Gruppen besitzen oder wenn bei höheren Temperaturen gearbeitet werden muß, wobei ein Teil des Kondensationsmittels durch Reaktion mit dem Lösungsmittel verbraucht wird.

Die Verbindungen der Formel (II) erhält man beispielsweise, indem man eine Verbindung der Formel

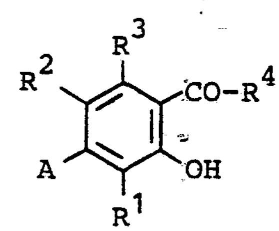

(III)

in der

$R^1$,$R^2$,$R^3$,$R^4$ und A die oben angegebene Bedeutung haben,

mit einer Verbindung der Formel

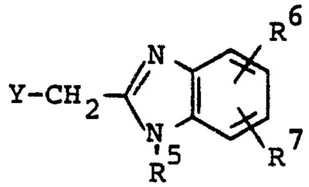

(IV)

in der

$R^5$, $R^6$ und $R^7$ ebenfalls die oben angegebene Bedeutung haben und

y Chlor oder Brom bedeutet,

in Gegenwart einer schwachen Base umsetzt.

Dabei wird zweckmäßigerweise so vorgegangen, daß man die Verbindungen der Formeln (III) und (IV) in äquimolaren Mengen in einem organischen Lösungsmittel bei 20-120°C, bevorzugt bei 40-90°C, in Gegenwart von mindestens molaren Mengen einer Base umsetzt.

Als Lösungsmittel zur Durchführung dieser Reaktion eignen sich z.B. Alkohole wie Methanol, Äthanol oder Äthylenglykolmonomethyläther, Ketone, wie z.B. Aceton, Methyläthylketon oder Methylisobutylketon, Fettsäureamide wie z.B. Dimethylformamid oder Dimethylacetamid, Sulfoxyde wie Dimethylsulfoxyd und Hexamethylphosphorsäuretriamid.

Als schwache Basen, die als säurebindende Mittel fungieren, können anorganische und organische Basen, bevorzugt Alkali- und Erdalkalicarbonate und -bicarbonate, beispielsweise Natriumcarbonat und Kaliumcarbonat, sowie tertiäre Amine, wie Triäthylamin oder Pyridin, eingesetzt werden.

Die Verbindungen der Formel (III) sind bekannt bzw. nach literaturbekannten Verfahren leicht zugänglich (siehe z.B. H. Giesecke und J. Hocker, Liebigs Annalen der Chemie, 1978, 345 sowie DE-OS 2 238 628 = US-PS 3 864 333).

Die Verbindungen der Formel (IV) werden nach dem in der US-PS 3 313 824 beschriebenen Verfahren durch Umsetzung von Halogenessigsäuren mit entsprechend substituierten o-Phenylendiaminen hergestellt.

Das erfindungsgemäße Verfahren kann besonders vorteilhaft auch als Eintopfreaktion durchgeführt werden.

Hierbei wird zunächst eine Verbindung der Formel (III) mit einer Verbindung der Formel (IV) in Gegenwart der oben beschriebenen säurebindenden Mittel, bevorzugt Natrium- oder Kaliumcarbonat bei Temperaturen zwischen 20 und 120°C, bevorzugt bei 40-90°C zu den Phenoläthern der Formel (II) umgesetzt, die anschließend direkt ohne jede Zwischenisolisierung mit starken Basen im obengenannten Temperaturbereich zu Verbindungen der Formel (I) cyclisiert werden. Die Produkte können in sehr guter bis quantitativer Ausbeute mit hoher Reinheit isoliert werden.

Die bisher in der Literatur beschriebenen Verfahren zur Herstellung von 2-[Benzimidazol-2-yl]-benzofuranen (siehe z.B. DE-OS 2 031 774 oder DE-OS 2 159 469) gehen von den Derivaten der entsprechenden Benzofuran-2-carbonsäuren aus, die mit entsprechend substituierten o-Phenylendiaminen oder o-Nitroanilinen in einer oder meist mehreren Stufen zu den gewünschten Produkten cyclisiert werden. Gegenüber diesem Verfahren bietet die erfindungsgemäße Arbeitsweise den Vorteil der leichteren Zugänglichkeit der Ausgangsverbindungen, der einfacheren technischen Durchführbarkeit der Reaktion, der höheren Ausbeute und der höheren Reinheit der Produkte.

Die nach dem erfindungsgemäßen Verfahren hergestellten Produkte sind z.T. bekannte wertvolle optische Aufheller (vgl. DE-OS 2 031 774).

Bevorzugte Verfahrensprodukte entsprechen der Formel (I) worin

$R^1$,$R^2$,$R^3$ Wasserstoff, Methyl, Äthyl, Methoxy, Chlor

$R^4$ Wasserstoff, Alkyl mit 1 bis 4 C-Atomen, vorzugsweise Methyl oder gegebenenfalls mit Methyl und/oder Methoxy substituiertes Phenyl,

$R^5$ Alkyl mit 1-4 C-Atomen, vorzugsweise Methyl, Hydroxyalkyl mit 2 bis 4 Kohlenstoffatomen, Cyanäthyl, gegebenenfalls durch Chlor, Methyl oder Methoxy substituiertes Phenyl, Cyclohexyl oder Benzyl,

$R^6$ Wasserstoff, Alkyl mit 1 bis 4 C-Atomen, vorzugsweise Methyl, Methoxy, Chlor, Alkylsulfonyl mit 1 bis 4 C-Atomen, Cyan, COOH, $SO_3H$, $COOR_5$, $SO_3R_5$ oder $CON(R_4)_2$

$R^7$ Wasserstoff, Methyl, Methoxy oder Chlor

A Wasserstoff, Chlor, $C_1$-$C_4$-Alkyl, $C_1$-$C_4$-Alkoxy, durch $C_1$-$C_4$-Alkyl und/oder $C_1$-$C_4$-Alkoxy substituiertes Phenyl oder ein Substituent der allgemeinen Formel

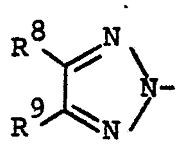

in der

$R^8$ $C_1$-$C_4$-Alkyl, $C_1$-$C_4$-Alkoxy, ein Phenylrest, der gegebenenfalls durch $C_1$-$C_4$-Alkyl, Phenyl, $C_1$-$C_4$-Alkoxy oder Chlor substituiert sein kann, Benzyl oder Styryl,

$R^9$ Wasserstoff, Cyan, Carboxy, $C_1$-$C_4$-Alkylcarbonylamino, Benzoylamino oder $R^8$

bedeuten.

Beispiel 1

7,2 g 4-(4-Methyl-5-phenyl-1,2,3-triazol-2-yl)-salicylaldehyd, 4,5 g 2-Chlormethyl-1-methylbenzimidazol und 3,2 g wasserfreies Natriumcarbonat werden in 50 ml Dimethylformamid 5 Stunden bei 70°C gerührt. Die Mischung wird anschließend auf 20° gekühlt und 3,2 g Kalium-tert.-butylat (ca. 95 %) zugegeben. Man rührt 5 Stunden bei Raumtemperatur nach, versetzt mit 50 ml Wasser, saugt ab, wäscht den Nutschkuchen mit Wasser neutral und trocknet bei 80°. Man erhält 9,7 g (96 % der Theorie) des Benzimidazols der Formel

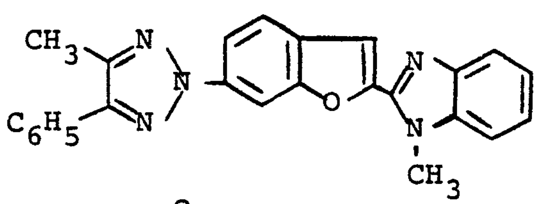

(V)

vom Fp. 222-224°C. Aus Toluol umkristallisiert schmilzt das Produkt bei 226°C. Das intermediär entstehende Zwischenprodukt der Formel

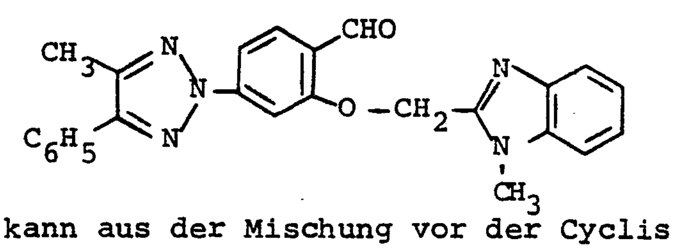

(VI)

kann aus der Mischung vor der Cyclisierung mit Kalium-tert.-butylat durch Ausgießen auf Wasser isoliert werden. Aus Methylglykol umkristallisiert schmilzt es bei 236°C.

Beispiel 2

14,7 g 2-Hydroxy-4-(4-methyl-5-phenyl-1,2,3-triazol-2-yl)-acetophenon, 9 g 2-Chlormethyl-1-methylbenzimidazol und 5,8 g wasserfreies Natriumcarbonat werden in 200 ml Dimethylformamid 7 Stunden bei 70° gerührt. Anschließend kühlt man auf 20°C, setzt 6,5 g Kalium-tert.-butylat (ca. 95 %) zu und rührt 5 Stunden bei Raumtemperatur. Nach dem Ausgießen auf 300 ml Wasser wird mit konzentrierter Salzsäure sauer gestellt, abgesaugt, neutral gewaschen und getrocknet. Man erhält 16 g (73 % der Theorie) Rohprodukt der Formel

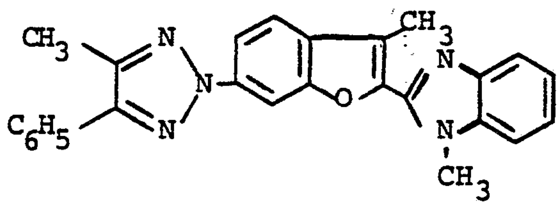

(VII)

vom Fp. 205-8°C. Aus Toluol umkristallisiert schmilzt die Verbindung bei 215-6°C.

Das 2-Hydroxy-4-(4-methyl-5-phenyl-1,2,3-triazol-2-yl)-acetophenon gewinnt man auf folgendem Wege:
56,4 g 3-/4-Methyl-5-phenyl-1,2,3-triazol-2-yl/-phenol werden in 200 ml Essigsäureanhydrid 30 Minuten auf 135-40°C erhitzt. Das überschüssige Essigsäureanhydrid wird im Vakuum abdestilliert und der Rückstand aus 150 ml Alkohol umkristallisiert. Man erhält 48,9 g (85 % der Theorie) Essigsäure-/3-(4-methyl-5-phenyl-1,2,3-triazol-2-yl)-phenyl/-ester vom Fp. 85°C. 117,2 g

dieses Esters werden mit 117,2 g pulverisiertem Aluminiumchlorid vermischt und langsam auf 140° erhitzt. Man rührt die Schmelze 1 Stunde bei 140°C, zerkleinert die erstarrte Schmelze und rührt sie in 800 ml 10 proz. Salzsäure ein, saugt ab, wäscht neutral und trocknet. Nach dem Umkristallisieren aus Glykolmonomethyläther/A-Kohle erhält man 54,6 g (46,7 % der Theorie) des gewünschten Acetophenons vom Fp. 167°C.

Beispiel 3

Unter Einsatz der Salicylaldehyde der Formeln

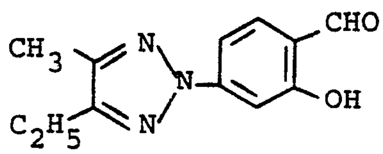

(VIII)

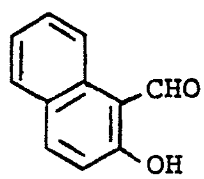

(IX)

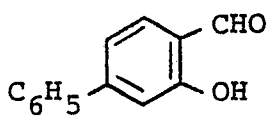

(X)

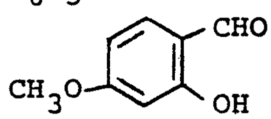

(XI)

und

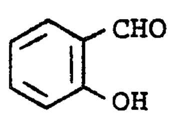

(XII)

werden bei Verwendung von 2-Chlormethyl-1-methyl-benzimidazol in Analogie zu den Beispielen 1 und 2 folgende Verbindungen erhalten.

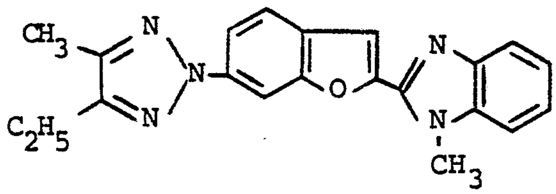

(XIII)

Fp. 180°C
Ausbeute: 76 % der Theorie

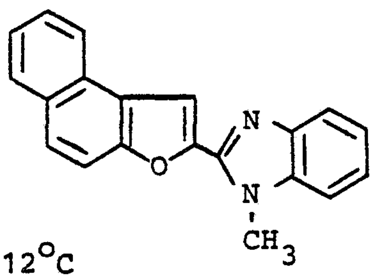

(XIV)

Fp. 212°C
Ausbeute: 79 %

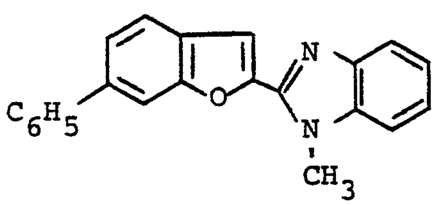

(XV)

Fp. 218°C
Ausbeute: 86 % der Theorie

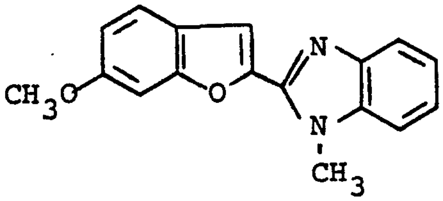

(XVI)

Fp. 251°C
Ausbeute: 77 % der Theorie

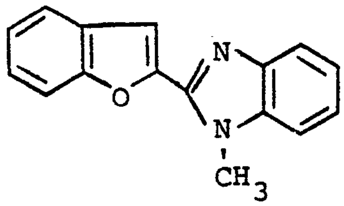

(XVII)

Fp. 231°C
Ausbeute: 72 % der Theorie

Beispiel 4

3,8 g 4-Methoxy-salicylaldehyd, 6,5 g 2-Chlormethyl-1-methyl-5-methylsulfonylbenzimidazol und 2,25 g wasserfreies Natriumcarbonat werden in 80 ml Dimethylformamid 7 Stunden bei 70°C gerührt. Nach dem Abkühlen auf 20°C gibt man 3,2 g Kalium-tert.-butylat (ca. 95 %) zu und rührt 5 Stunden bei Raumtemperatur. Anschließend wird die Suspension auf 250 ml Wasser gegossen, mit konzentrierter Salzsäure sauer gestellt, abgesaugt, neutral gewaschen und getrocknet. Man erhält 7,4 g (83 % der Theorie) Rohprodukt der Formel

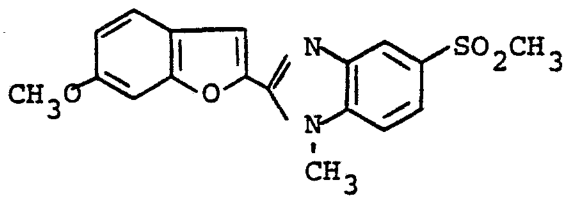

(XVIII)

Nach dem Umkristallisieren aus Toluol schmilzt die Verbindung bei 205°C.

Verwendet man anstelle von 4-Methoxysalicylaldehyd die äquivalente Menge 4-(4-Methyl-5-phenyl-1,2,3-triazol-2-yl)-salicylaldehyd und verfährt im übrigen wie vorstehend beschrieben, so erhält man die Verbindung der Formel

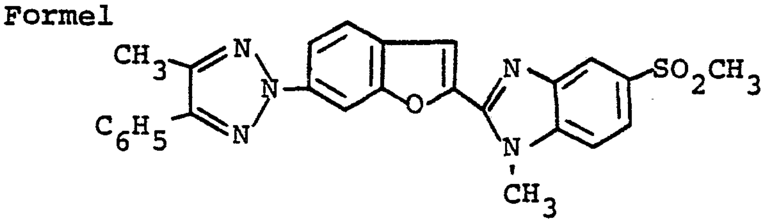

(XIX)

Fp. 269-70°C
Ausbeute: 98 % der Theorie.

Beispiel 5

Verwendet man anstelle von 6,5 g 2-Chlormethyl-1-methyl-5-methylsulfonylbenzimidazol die äquivalente Menge 5-Chlor-2-chlormethyl-1-methylbenzimidazol und verfährt

im übrigen wie in Beispiel 4 beschrieben, so erhält man 6,8 g (87 % der Theorie) der Verbindung der Formel

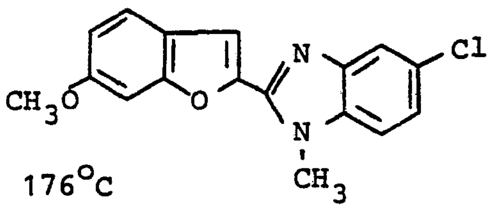

(XX)

Fp. 176°C

Unter Einsatz von 4-(4-Phenyl-5-methyl-1,2,3-triazol-2-yl)-salicylaldehyd, 2-Hydroxy-4-(4-methyl-5-phenyl-1,2,3-triazol-2-yl)-acetophenon, 4-(4-Äthyl-5-methyl-1,2,3-triazol-2-yl)-salicylaldehyd werden bei Verwendung von 5-Chlor-2-chlormethyl-1-methyl-benzimidazol in Analogie zu Beispiel 4 folgende Verbindungen erhalten:

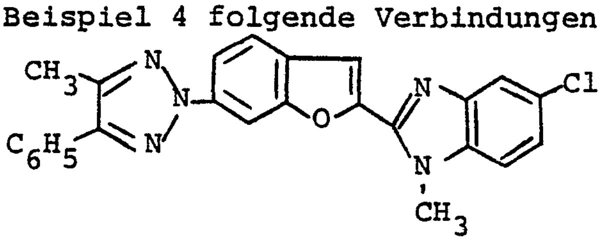

(XXI)

Fp. 230-1°C
Ausbeute: 93 % der Theorie

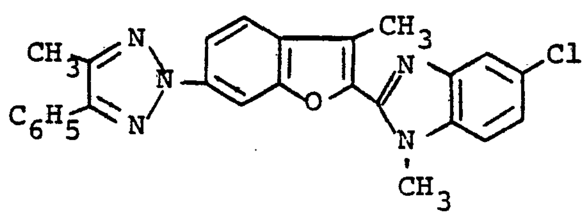

(XXII)

Fp.: 248°C
Ausbeute: 84 % der Theorie

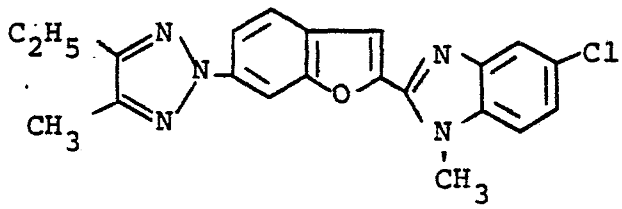

(XXIII)

Fp. 196°C
Ausbeute: 88 % der Theorie

Beispiel 6

Verwendet man anstelle von 6,5 g 2-Chlormethyl-1-methyl-5-methylsulfonylbenzimidazol die äquivalente Menge 2-Chlormethyl-1,5-dimethylbenzimidazol und verfährt im übrigen wie in Beispiel 4 beschrieben, so erhält man 5,1 g (70 % der Theorie) der Verbindung der Formel

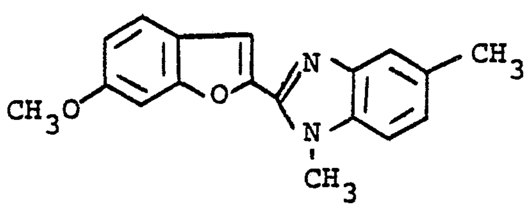

(XXIV)

Fp. 246°C

Setzt man 4-Phenylsalicylaldehyd, 4-(4-Phenyl-5-methyl-1,2,3-triazol-2-yl)-salicylaldehyd bzw. 2-Hydroxy-4-(4-methyl-5-phenyl-1,2,3-triazol-2-yl)-acetophenon in Analogie zu Beispiel 4 mit 2-Chlormethyl-1,5-dimethylbenzimidazol um, so erhält man folgende Verbindungen:

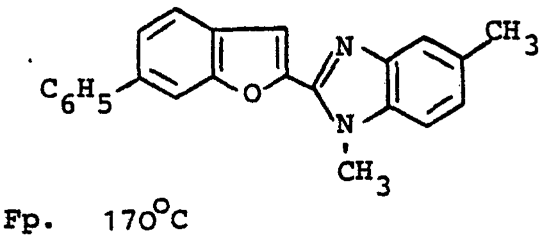

(XXV)

Fp. 170°C

Ausbeute: 83 % der Theorie

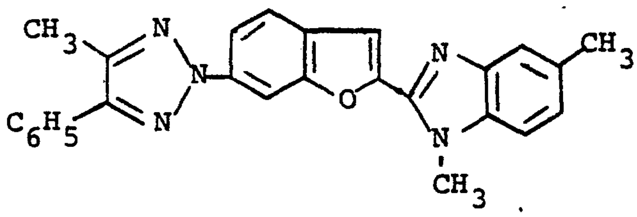

(XXVI)

Fp. 220°C

Ausbeute: 80 % der Theorie

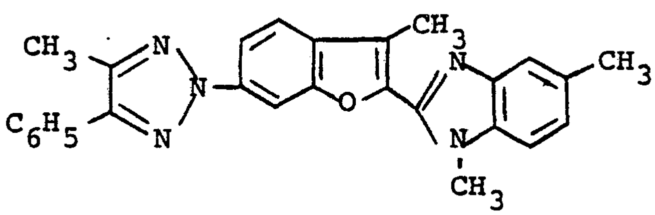

(XXVII)

Fp. 253°C

Ausbeute: 86 % der Theorie

Patentansprüche

1. Verfahren zur Herstellung von 2-/Benzimidazol-2-yl/-benzofuranen der allgemeinen Formel

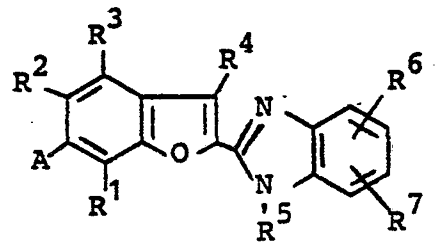

(I)

in der

| | |
|---|---|
| $R^1$, $R^2$, $R^3$ | Wasserstoff, Alkyl, Alkoxy oder Halogen |
| $R^4$ | Wasserstoff, Aryl, Alkyl |
| $R^5$ | Wasserstoff, Alkyl, Cycloalkyl, Alkenyl, Aryl oder Aralkyl |
| $R^6$ | Wasserstoff, Halogen, Alkyl, Alkoxy, Alkylsulfonyl, Phenylsulfonyl, CN, $CF_3$, COOR, $SO_3R$, CONRR' oder $SO_2NRR'$ |
| $R^7$ | Wasserstoff, Halogen, Alkyl, Alkoxy, |
| A | Wasserstoff, Halogen, Alkyl, Alkoxy oder Aryl oder einen Substituenten der allgemeinen Formel |

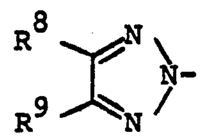

in der

$R^8$ Aryl, Alkyl, Aralkyl, Styryl oder Alkoxy,

$R^9$ Wasserstoff, CN, COOR, CONRR' oder $R^8$

$R^8$ und $R^9$ zusammen einen annellierten hydroaromatischen oder aromatischen Ring.

R Alkyl und

R' R oder H bedeuten, wobei die vorstehend genannten Kohlenwasserstoff- und Alkoxyreste sowie die gegebenenfalls annellierten Ringsysteme durch in der Aufhellerchemie übliche Substituenten substituiert sein können,

dadurch gekennzeichnet, daß man Verbindungen der Formel

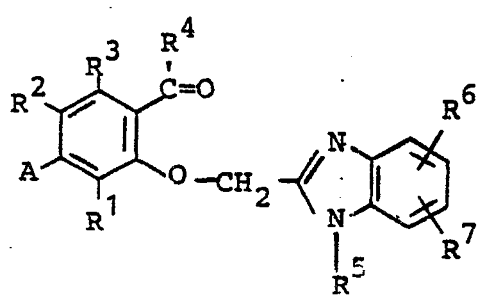

(II)

in der

$R^1,R^2,R^3,R^4,R^5,R^6,R^7$ und A die oben angegebene Bedeutung haben,

mit stark basischen Kondensationsmitteln ringschließend umsetzt.

2. Verfahren gemäß Anspruch 1, dadurch gekennzeichnet, daß man Verbindungen der Formel

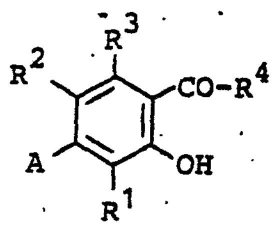

(III)

in der

$R^1,R^2,R^3,R^4$ und A die oben angegebene Bedeutung haben

mit Verbindungen der Formel

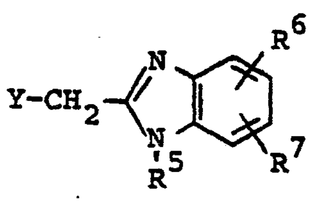

(IV)

in der

$R^5, R^6, R^7$ die oben angegebene Bedeutung haben und

Y Chlor oder Brom bedeutet,

in Gegenwart einer schwachen Base zu Verbindungen der Formel (II) umsetzt und diese ohne Zwischenisolierung gemäß Anspruch 1 cyclisiert.

Europäisches Patentamt

# EUROPÄISCHER RECHERCHENBERICHT

0012291

Nummer der Anmeldung

EP 79 10 4802

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | DE - A - 2 400 656 (HOECHST)<br>* Seiten 11,12 *<br>-- | 1 |
| P | EP - A - 0 005 465 (BAYER)<br>* Seiten 44-50 *<br>---- | 1 |

KLASSIFIKATION DER ANMELDUNG (Int.Cl.[3])

C 07 D 405/14
405/04//
C 07 D 403/12
249/06

RECHERCHIERTE SACHGEBIETE (Int. Cl.[3])

C 07 D 405/14
405/04

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 26-02-1980 | DE BUYSER |